# EUROPÄISCHE PATENTSCHRIFT

(11) **EP 3 050 634 B1**
(45) Veröffentlichungstag und Bekanntmachung des Hinweises auf die Patenterteilung: **21.11.2018**
(21) Anmeldenummer: 16151197.7
(22) Anmeldetag: 14.01.2016
(51) Int. Cl.: B05C 17/005, A61B 17/88, B65D 81/32, B05C 17/015

(54) **PASTEN-APPLIKATIONSVERFAHREN UND VORRICHTUNG ZUM MISCHEN EINER PASTE AUS ZWEI KOMPONENTEN**
PASTE APPLICATION METHOD AND DEVICE FOR THE MIXING OF A PASTE FROM TWO COMPONENTS
PROCÉDÉ ET SYSTÈME D'APPLICATION DE PÂTE DESTINÉ AU MÉLANGE D'UNE PÂTE À PARTIR DE DEUX COMPOSANTS

(30) Priorität: 27.01.2015 DE 102015101126
(43) Veröffentlichungstag der Anmeldung: 03.08.2016
(73) Patentinhaber: Heraeus Medical GmbH, 61273 Wehrheim (DE)
(72) Erfinder: Dr. Vogt, Sebastian, 99092 Erfurt (DE)
(74) Vertreter: Heraeus IP

(56) Entgegenhaltungen:
- EP-A- 0 213 073
- DE-T2- 69 415 293
- DE-U1- 7 934 535
- US-A- 4 366 919

## Beschreibung

Die Erfindung betrifft eine Pasten-Applikationsvorrichtung zum Lagern zweier Ausgangskomponenten, zum Mischen der Ausgangskomponenten zu einer Paste und zum Applizieren der Paste.

Die Erfindung betrifft ferner ein Verfahren zum Mischen und Austreiben einer Paste.

Gegenstand der Erfindung ist somit eine manuell zu bedienende Pasten-Applikationsvorrichtung, die zum Austragen von pastenförmigen Massen, insbesondere von pastenförmigem Polymethylmethacrylat-Zementteig (PMMA-Zementteig) bestimmt ist. Die Pasten-Applikationsvorrichtung und das Verfahren sind zudem zum Lagern, Vermischen und zum Austragen von pastenförmigen Zweikomponentensystemen vorgesehen.

Konventionelle Polymethylmethacrylat-Knochenzemente (PMMA-Knochenzemente) werden meist aus einer pulverförmigen Komponente und einer flüssigen Monomerkomponente hergestellt (K.-D. Kühn: Knochenzemente für die Endoprothetik: ein aktueller Vergleich der physikalischen und chemischen Eigenschaften handelsüblicher PMMA-Zemente. Springer-Verlag Berlin Heidelberg New York, 2001). Diese PMMA-Knochenzemente werden nach Vermischung des Zementpulvers mit der flüssigen Monomerkomponente im noch nicht ausgehärteten, pastenförmigen Zustand als Zementteig appliziert. Bei Verwendung von Mischsystemen befindet sich der Zementteig im Fall von Pulver-Flüssigkeitszementen in einer Kartusche. Aus dieser Kartusche wird der Zementteig durch Bewegung eines Förderkolbens heraus gedrückt. Eine solche Bewegung des Förderkolbens kann mit Hilfe einer mechanischen Applikationsvorrichtung bewirkt werden.

Bei pastenförmigen Zweikomponenten-Pasten beziehungsweise Zweikomponenten-Knochenzementen, wie beispielsweise aus der DE 10 2007 050 762 B3, der DE 10 2008 030 312 A1 oder der DE 10 2007 052 116 B4 bekannt, werden beide pastenförmigen Komponenten in zwei separaten Kartuschen mit zwei separaten Förderkolben aufbewahrt. Bei der Applikation werden beide Pasten durch Bewegung der Förderkolben aus den Kartuscheninnenräumen in einen statischen Mischer gepresst und nach erfolgter Vermischung durch ein Austragsrohr ausgetragen.

Die Applikation von pastenförmigen Kleb- und Dichtstoffen erfolgt in der prinzipiell gleichen Weise mit Pasten-Applikationsvorrichtungen.

Zum Auspressen von zähen viskosen Massen werden gegenwärtig Pasten-Applikationsvorrichtungen verwendet, die manuell oder pneumatisch oder auch elektrisch angetrieben werden. Übliche einfache mechanische Pasten-Applikationsvorrichtungen nutzen insbesondere Klemmstangen zum Auspressen, die durch einen manuell zu betätigenden Kipphebel angetrieben werden. Bei hochviskosen Pasten sind diese Vorrichtungen nur mit sehr großem Kraftaufwand zu bedienen. Dieser Kraftaufwand ist medizinischen Anwendern im OP nicht zu zumuten. Elektrisch angetriebene Auspressvorrichtungen können sowohl mit Akkumulatoren beziehungsweise mit Batterien als auch mit Hilfe einer stationären Stromversorgung angetrieben werden. Diese Vorrichtungen können mit ihren zum Teil sehr großen Kräften besonders zähe pastenförmige Massen auspressen. Nachteilig ist jedoch bei der Verwendung von Elektromotoren, dass diese Buntmetalle enthalten und kostenintensiv sind. Zudem müssen Akkumulatoren vorgehalten werden oder es ist ein im OP-Bereich hinderlicher Kabelanschluss vorzusehen, mit dem die Pasten-Applikationsvorrichtung an ein Stromnetz angeschlossen sein muss.

Pneumatische Pasten-Applikationssysteme, wie beispielsweise die aus EP 0 213 073; der US 4,366,919; der US 2004 074 927 A1 oder der US 6 935 541 B1 bekannten Systeme, erfordern einen Druckluftanschluss. Dazu sind Druckluftschläuche notwendig, welche die Bewegung des Anwenders und die Anwendung des Pasten-Applikationssystems behindern können. Alternativ dazu ist auch die Verwendung von Druckgaspatronen zur Bereitstellung von Druckgas möglich. Exemplarisch dafür seien die Dokumente DE 79 34 535 U1; US 2 818 999 A, und EP 1 118 313 A1 genannt.

Ein interessanter Vorschlag ist in der US 4 925 061 A1 beschrieben. Bei diesem System ist keine Gaspatrone in der Vorrichtung angeordnet. Die Vorrichtung muss über ein Ventil vor der Anwendung mit Druckgas aufgefüllt werden. In dieser Vorrichtung ist eine Kartusche enthalten, in der die viskose Masse angeordnet ist. Hinter der viskosen Masse ist ein Kolben angeordnet, der mit einem Faltenbalg mit einem Ende der Kartusche verbunden ist. In dem Hohlraum, der von dem Kolben, dem Faltenbalg und dem Kartuschenende gebildeten wird, wird vor der Anwendung flüssiges Gas durch ein Ventil eingefüllt. Dieser Vorschlag ist kritisch zu sehen, weil bei der Expansion von Flüssiggas eine Abkühlung eintritt, die zur Versprödung des elastischen Faltenbalgs führen kann. Bei einer Versprödung des Faltenbalgs sind Undichtigkeiten nicht auszuschließen. Dadurch ist es dann möglich, dass Druckgas durch den Faltenbalg austritt und neben dem Kolben in die viskose Masse eintritt. Bei Polymethylmethacrylat-Knochenzementpasten ist eine Vermischung der Pasten mit Druckgas nicht akzeptabel. Gasblasen würden zu einer Schwächung des ausgehärteten Polymethylmethacrylat-Knochenzementes führen. Weiterhin ist das Befüllen mit flüssigem Druckgas kompliziert und medizinischen Anwendern im OP nicht zu zumuten.

Mit der DE 10 2010 019 223 B4 wurde eine Zementiervorrichtung vorgeschlagen, bei der durch Bewegung eines Drehventils eine Druckgaspatrone angestochen wird. Das Druckgas drückt direkt auf die Kolben der Vorrichtung. Der Austrag der Pasten wird durch ein Ventil reguliert. Nachteilig ist an der Vorrichtung, dass hochfeste Kunststoffe für die Kartuschen verwendet werden müssen, weil kein Druckbehälter vorgesehen ist.

Grundsätzlich kann es bei allen druckgasgetriebenen Pasten-Applikationsvorrichtungen mit Förderkolben passieren, dass sich Druckgas ungewollt an den Förderkolben vorbei in die Pasten presst. Je höher der Gasdruck ist, desto stärker kann sich dieses Problem ausprägen.

Weiterhin sind aus dem medizinischen Bereich Vorrichtungen zum Austrag von Medikamenten bekannt, die auch Druckgaspatronen als Energiequelle nutzen, bei denen jedoch keine Regulierung des Volumenstroms des Medikaments über Ventile vorgesehen ist (US 2008 086 079 A1, US 2008 208 114 A1). Solche Systeme sind für Knochenzemente nicht gut verwendbar, da sie keine gezielte Portionierung des Knochenzementteigs während der OP erlauben.

Die Aufgabe der Erfindung besteht darin, die Nachteile des Stands der Technik zu überwinden. Insbesondere soll eine Pasten-Applikationsvorrichtung und ein Verfahren zum Mischen und Austreiben einer Paste gefunden werden, mit dem auch sehr zähe Ausgangskomponenten mit einem einfachen Aufbau gelagert, gemischt und ausgetragen werden können. Dabei soll das Pasten-Applikationssystem ausreichend kostengünstig sein, um eine einmalige Anwendung (Wegwerfartikel) zu ermöglichen. Wegwerfartikel sind aufgrund der hohen hygienischen Anforderungen im OP-Bereich besonders vorteilhaft.

Die Aufgabe der Erfindung besteht darin, die Nachteile der bisher bekannten Pasten-Applikationsvorrichtungen zum Lagern, Vermischen und Applizieren von pastenförmigen Polymethylmethacrylat-Knochenzementen zu überwinden, bei denen die Energie zum Vermischen und Applizieren von einer Druckgaspatrone bereitgestellt wird, die in der Vorrichtung integriert ist.

Eine Aufgabe der Erfindung ist dabei ferner darin zu sehen, eine Pasten-Applikationsvorrichtung bereit zu stellen, die ohne Verwendung von externen stationären Energiequellen, wie Druckluft oder elektrischen Strom, angetrieben werden kann und die in der Lage ist, zähe pastöse Massen auszupressen und zu mischen. Die Pasten-Applikationsvorrichtung soll auch kein Kupfer und auch keine Kupferlegierungen enthalten. Sie soll ferner manuell durch den Anwender ortsunabhängig angewendet werden können und einen maximal vereinfachten Aufbau besitzen. Dadurch soll es möglich sein, eine kostengünstige Pasten-Applikationsvorrichtung bereit zu stellen, die nur zum einmaligen Gebrauch bestimmt ist. Weiterhin soll die Erfindung auch die Aufgabe lösen, ein Verfahren zum Austragen von pastenförmigen Massen mit der zu entwickelnden Pasten-Applikationsvorrichtung zu entwickeln.

In der Pasten-Applikationsvorrichtung soll weiterhin vor der Zementapplikation eine separate Lagerung von zwei Zementpasten möglich sein. Die Oberfläche der Pasten-Applikationsvorrichtung soll weiterhin mit Ethylenoxid zu sterilisieren sein. Wichtig ist, dass während der Applikation durch den erforderlichen hohen Druck keine unerwünschten Zementaustritte möglich sind, die zur Kontamination des medizinischen Anwenders und des Operations-Saals (OP-Saals) führen könnten. Die Pasten-Applikationsvorrichtung sollte möglichst mit geringem Kraftaufwand manuell zu betätigen sein. Für die Anwendung ist es weiterhin wichtig, dass beim Stoppen der Bedienung der Pasten-Applikationsvorrichtung, der Zementteig beziehungsweise die Paste nicht in größeren Mengen nachfließt.

Es soll ferner eine Vorrichtung entwickelt werden, die zum Lagern, Vermischen und Austragen von hochviskosen pastenförmigen Polymethylmethacrylat-Knochenzement geeignet ist, wobei als Energiequelle Druckgas verwendet werden soll, das in einer Druckgaspatrone gelagert wird, die in der Pasten-Applikationsvorrichtung integriert ist. Eine weitere Aufgabe besteht darin, dass die Pasten-Applikationsvorrichtung hohe Gasdrücke von mehr als 30 bar über einen Anwendungszeitraum von wenigen Minuten sicher mechanisch toleriert, ohne dass sich die Pasten-Applikationsvorrichtung deformiert oder zerstört wird. Die zu entwickelnde Pasten-Applikationsvorrichtung soll zudem eine kostengünstige Fertigung erlauben. Weiterhin soll die Pasten-Applikationsvorrichtung möglichst keine den Druckgasfluss regulierende Ventile enthalten und auch keine separaten Vorrichtungen, die zur Verdampfung von flüssigem Druckgas bestimmt sind. Die zu entwickelnde Pasten-Applikationsvorrichtung muss so robust aufgebaut sein, dass das Druckgas mit verflüssigtem Druckgas vermischt sein kann. Bei Verwendung von flüssigem Kohlendioxid als Druckgas muss die Pasten-Applikationsvorrichtung auch Gemische aus gasförmigen, flüssigen und auch festem Kohlendioxid ohne mechanische Beschädigung oder Deformierung tolerieren.

Die Aufgaben der Erfindung werden gelöst durch eine Pasten-Applikationsvorrichtung zum Lagern zweier Ausgangskomponenten, zum Mischen der Ausgangskomponenten zu einer Paste und zum Applizieren der Paste aufweisend
eine Zweikomponentenkartusche aufweisend zwei zylindrische Innenräume, zwei axial in den Innenräumen verschiebbare Förderkolben, die die Innenräume auf einer ersten Seite der Zweikomponentenkartusche begrenzen, und zumindest zwei Austragsöffnungen, durch die die Innenräume an einer der ersten Seite gegenüberliegenden zweiten Seite der Zweikomponentenkartusche geöffnet sind,
einen Hohlzylinder aus einem Kunststoff mit einer offenen Stirnseite und einer bereichsweise geschlossenen Stirnseite, in dem sich ein axial beweglicher Kolben mit zwei daran befestigten Stößeln befindet, wobei die Stößel in Richtung der offenen Stirnseite ausgerichtet sind, wobei der Hohlzylinder mit der offenen Stirnseite axial an der ersten Seite der Zweikomponentenkartusche anliegend angeordnet ist und wobei der Kolben gasdicht mit den Innenwänden des Hohlzylinders abschließt,
wobei die Zweikomponentenkartusche und der Hohlzylinder in einem Druckbehälter aus Metall, einem hochfesten Kunststoff oder einem faserverstärkten Kunststoff angeordnet sind, wobei der Druckbehälter an dem Hohlzylinder anliegt oder einen Abstand von höchstens 0,1 mm zum Hohlzylinder aufweist,
wobei der Hohlzylinder an der bereichsweise geschlossenen Stirnseite einen Anschluss für eine Druckgaspatrone aufweist, der sich durch eine Öffnung im Druckbehälter erstreckt und
wobei in Fließrichtung hinter den Austragsöffnungen ein manuell bedienbares Ventilelement befestigt ist oder ein Austragsrohr mit einem manuell bedienbaren Ventilelement zu befestigen ist, wobei mit dem hinter den Austragsöffnungen befestigten Ventilelement der Volumenfluss der Ausgangskomponenten durch die Austragsöffnungen zu regulieren ist.

Dass der Volumenfluss der Ausgangskomponenten durch die Austragsöffnungen zu regulieren ist, bedeutet insbesondere, dass der Volumenfluss durch schließen des Ventilelements zu stoppen ist.

Unter einer Zylindergeometrie wird erfindungsgemäß die allgemeine Zylinderform mit beliebiger Grundform verstanden, also nicht nur Zylinder mit kreisförmiger Grundfläche.

Die Innenräume sind vorzugsweise mit zwei zu mischenden Ausgangskomponenten gefüllt, von denen zumindest eine eine Paste ist, wobei besonders bevorzugt die gemischten Ausgangskomponenten einen PMMA-Knochenzement bilden. Das Gemisch soll mit der Pasten-Applikationsvorrichtung aufzutragen sein.

Vorzugsweise schließt der Kolben nicht nur gasdicht mit den Innenwänden des Hohlzylinders ab, sondern sogar druckdicht bis zumindest 10 Atmosphären beziehungsweise bis zumindest 1000 kPa, besonders bevorzugt druckdicht bis zumindest 3000 kPa.

Auch bei einem geringem Abstand von maximal 0,1 mm zwischen dem Druckbehälter und dem Hohlzylinder kann der Druckbehälter die Druckkräfte aufnehmen, da bei der Entspannung des Gases aus der Druckgaspatrone, die beim Einleiten des Druckgases aus der Druckgaspatrone in der Hohlzylinder erfolgt, eine Expansion (teilweise adiabatische Expansion) stattfindet, bei der das sich entspannende Gas abkühlt, diese die umgebenden Behältnisse abkühlen und sich infolge dessen der Hohlzylinder aus Kunststoff weniger stark zusammenzieht als der Druckbehälter, wenn dieser aus Metall oder Metalllegierung gefertigt ist und somit sich der metallische Druckbehälter an den Hohlzylinder anlegt.

Metalle oder Metalllegierungen sind als Material für den Druckbehälter bevorzugt, da viele metallische Werkstoffe trotz hoher Druckfestigkeit beziehungsweise trotz hoher Zugfestigkeit leicht zu bearbeiten sind. Als hochfeste Kunststoffe für den Druckbehälter kommen Duroplaste, Polyamide, Polyamide-co-imide, Polysulfone, Polyketone und Polyetherketone in Frage, die allerdings schwerer als Metalle oder Metalllegierungen zu bearbeiten sind und daher als Material für den Druckbehälter weniger bevorzugt sind. Als faserverstärkte Kunststoffe sind Glasfaser-verstärkte Kunststoffe besonders bevorzugt.

Bevorzugt ist ferner das Ventilelement außerhalb des Druckbehälters angeordnet. Die Achse des Ventilelements besteht erfindungsgemäß bevorzugt aus Stahl, um die Kräfte, die auf die Klappe wirken, ohne Verformung oder Zerstörung aufnehmen zu können.

Bei erfindungsgemäßen Pasten-Applikationsvorrichtungen kann auch vorgesehen sein, dass das Ventilelement in einer Leitung angeordnet ist, die durch ein Austragsrohr gebildet ist, wobei bevorzugt im Austragsrohr zusätzlich ein statischer Mischer vorgesehen ist, mit dem die Ausgangskomponenten beim Durchströmen durch den Mischer durchmischbar sind.

Hierdurch kann ein besonders einfacher Aufbau erreicht werden, der kostengünstig realisiert werden kann, der gleichzeitig für Fehlfunktionen unanfällig ist und der zum Regulieren der Ströme der Ausgangskomponenten mit großem Druck auch stabil genug ist.

Des Weiteren kann vorgesehen sein, dass der Druckbehälter zweiteilig aufgebaut ist, wobei die beiden Teile durch Vernietung, durch Verschraubung und/oder durch eine Überwurfmutter kraftschlüssig verbunden sind, wobei vorzugsweise ein erster Teil der beiden Teile des Druckbehälters die Zweikomponentenkartusche beinhaltet und der zweite der beiden Teile des Druckbehälters (also der andere Teil des Druckbehälters) den Hohlzylinder.

Hierdurch werden die Herstellung und die Montage der Pasten-Applikationsvorrichtung vereinfacht. Insbesondere die Befüllung der Zweikomponentenkartusche mit den Ausgangskomponenten wird hierdurch deutlich vereinfacht. Bevorzugt sind die beiden Teile des Druckbehälters formschlüssig und/oder stoffschlüssig miteinander verbunden.

Bevorzugte Ausgestaltungen der Erfindung können sich dadurch auszeichnen, dass die Zweikomponentenkartusche zumindest bereichsweise eine Koaxialkartusche ist, wobei in der Koaxialkartusche einer der Innenräume zylindrisch und innenliegend ist und der andere Innenraum zylindrisch ist und den innenliegenden Innenraum koaxial umschließt.

Die Förderkolben sind der Innenform der zylindrischen Innenräume angepasst. Die Zylinderform hat den Vorteil, dass sich die auftretenden großen Kräfte beim Auspressen der Ausgangskomponenten beziehungsweise beim Beaufschlagen der Zweikomponentenkartusche mit dem Druck aus der Druckgaspatrone gleichmäßig vom Druckbehälter aufgenommen werden können. Zudem können Bauteile mit zylindrischer Geometrie kostengünstig gefertigt werden.

Ferner kann vorgesehen sein, dass die Pasten-Applikationsvorrichtung einen Verschluss zum Verschließen der Austragsöffnungen aufweist, der mit einem Befestigungsmittel des Verschlusses, insbesondere mit einem Gewinde des Verschlusses, an einem Gegenbefestigungsmittel im Bereich der Austragsöffnungen, insbesondere an einem Gegengewinde im Bereich der Austragsöffnungen, zu befestigen ist, wobei vorzugsweise an dem Gegenbefestigungsmittel das Austragsrohr zu befestigen ist, das zu diesem Zweck ebenfalls ein dementsprechendes Befestigungsmittel aufweist.

Hierdurch können die Ausgangskomponenten länger in der Pasten-Applikationsvorrichtung gelagert werden, da mit dem Verschluss ein besserer und luftdichterer Abschluss der Innenräume mit den Ausgangskomponenten darin erreicht werden kann, als wenn nur das Ventilelement die Innenräume abdichten würde.

Dabei kann vorgesehen sein, dass der Verschluss ein Schlüssel zum Öffnen der Druckgaspatrone und/oder zum Verbinden der Druckgaspatrone mit dem Anschluss des Hohlzylinders ist, wobei vorzugsweise der Verschluss als Schlüssel bodenseitig auf die Druckgaspatrone zu stecken ist und damit die Druckgaspatrone zum Öffnen der Druckgaspatrone und/oder zum Verbinden der Druckgaspatrone mit dem Anschluss des Hohlzylinders bewegbar, insbesondere drehbar und/oder in Längsrichtung verschiebbar ist.

Hierdurch kann ein frühzeitiges versehentliches Öffnen der Druckgaspatrone vermieden werden, so dass die Bedienung der Pasten-Applikationsvorrichtung vereinfacht wird.

Bevorzugt kann auch vorgesehen sein, dass an dem Anschluss des Hohlzylinders ein Druckgaspatronenbefestigungsmittel vorgesehen ist, mit dem die Druckgaspatrone an dem Anschluss druckdicht zu befestigen ist, und/oder an dem Anschluss des Hohlzylinders ein Stechdorn zum Öffnen der Druckgaspatrone vorgesehen ist.

Durch diesen Aufbau wird eine sichere und dichte Verbindung erreicht beziehungsweise eine frühzeitige ungewollte Öffnung der Druckgaspatrone vermieden. Das Druckgaspatronenbefestigungsmittel ist vorzugsweise ein Innengewinde, in das ein Außengewinde der Druckgaspatrone eingeschraubt werden kann. An einer Verbindungsfläche kann zudem eine Dichtung (beispielsweise in Form eines O-Rings) vorgesehen sein, mit dem eine bessere Abdichtung der Verbindung der Druckgaspatrone zum Hohlzylinder erreicht wird.

Zur Verbesserung der Lagerungsmöglichkeit kann vorgesehen sein, dass zwischen dem Hohlzylinder und der Zweikomponentenkartusche eine Schutzfolie angeordnet ist, die die Innenräume der Zweikomponentenkartusche auf der ersten Seite verschließen, wobei die Schutzfolie vorzugsweise auf die Zweikomponentenkartusche aufgeklebt, aufgeschweißt oder angehaftet ist.

Mit der Schutzfolie kann eine bessere Abdichtung der Innenräume erreicht werden, so dass eine längere beziehungsweise qualitätswahrende Lagerung von Ausgangskomponenten mit flüchtigen Bestandteilen, wie beispielsweise Methylmethacrylat, in den Innenräumen möglich wird. Die Schutzfolie wird im Betrieb beim Vortreiben der Stößel durch den Kolben im Hohlzylinder durchstoßen, so dass die Stößel die Förderkolben in der Zweikomponentenkartusche vortreiben können und damit die Ausgangskomponenten aus der Zweikomponentenkartusche durch die Austragsöffnungen aus den Innenräumen der Zweikomponentenkartusche austreiben können.

Bevorzugt ist die Schutzfolie eine Aluminium-Verbundfolie. Aluminium beziehungsweise Aluminium-beschichtete Folien sind gegenüber Methylmethacrylat-Dämpfen besonders dicht, so dass die Pasten-Applikationsvorrichtung dann besonders gut zum Lagern der Ausgangskomponenten eines PMMA-Knochenzements geeignet ist.

Bei bevorzugten Pasten-Applikationsvorrichtungen kann vorgesehen sein, dass der Druckbehälter aus Aluminium, Zink, einer Aluminiumlegierung oder Stahl besteht.

Mit diesen Materialien können die auftretenden Kräfte durch den Druckbehälter gut aufgenommen werden, ohne dass es zu einer Zerstörung des Druckbehälters kommt. Zudem sind diese metallischen Werkstoffe bei der Herstellung gut in die gewünschte Form zu bringen.

Es kann erfindungsgemäß bevorzugt vorgesehen sein, dass der Druckbehälter ein Zug-Modul nach EN ISO 527 größer 1500 MPa aufweist.

Es kann ferner erfindungsgemäß bevorzugt vorgesehen sein, dass der Druckbehälter eine Druckfestigkeit von mindestens 3 MPa aufweist.

Es kann auch vorgesehen sein, dass der Druckbehälter einen zylindrischen Innenraum zur Aufnahme des Hohlzylinders und der Zweikomponentenkartusche aufweist. Die zylindrische Geometrie ist sowohl zur Aufnahme der Kräfte besonders gut geeignet, als auch in der Fertigung leicht herzustellen.

Besonders vorteilhafte Ausgestaltungen der vorliegenden Erfindung können vorsehen, dass das manuell bedienbare Ventilelement über ein Bedienelement von außen bedienbar ist, vorzugsweise über einen Hebel am Austragsrohr oder einen Abzug an einem Griff der Pasten-Applikationsvorrichtung bedienbar ist.

Hierdurch kann die Pasten-Applikationsvorrichtung von außen leicht bedient werden und ist dadurch anwenderfreundlich. Bevorzugt kann das Ventilelement im Austragsrohr von außen zu betätigen sein, wobei das Ventilelement besonders bevorzugt durch eine manuell ausgelöste Drehbewegung geöffnet und geschlossen wird. Auch kann vorgesehen sein, dass ein Verschlussteil des Ventilelements mit einem Hebel verbunden ist, der an der Außenseite der Pasten-Applikationsvorrichtung parallel zur Längsachse angeordnet ist, wobei der Hebel zum Öffnen und Schließen des Ventilelements manuell bewegt wird, so dass sich das Verschlussteil im Ventilsitz dreht. Ferner kann vorgesehen sein, dass das Verschlussteil kraftschlüssig oder formschlüssig mit einer manuellen Abzugsvorrichtung verbunden ist, die parallel zur Längsachse der Pasten-Applikationsvorrichtung angeordnet ist, wobei bei manueller linearer Bewegung der Abzugsvorrichtung parallel zur Längsachse oder bei einer manueller Kippbewegung der Abzugsvorrichtung, die tangential zur Längsachse erfolgt, die Bewegung über ein Getriebe, insbesondere ein Koppelgetriebe, in eine zu mindestens partielle Drehbewegung des Verschlussteils des Ventilelements überführt wird. Bei einer Pasten-Applikationsvorrichtung mit Abzugsvorrichtung kann auch vorgesehen sein, dass das Verschlussteil des Ventilelements mit einem ersten Hebel verbunden ist, wobei ein Hebelende als erste schiefe Ebene mit einem Winkel von 20° bis 45° ausgebildet ist, und dieser ersten schiefen Ebene gegenüber eine zweite schiefe Ebene eines zweiten Hebels, der mit einem manuell zu betätigenden Abzug verbunden ist, angeordnet ist, wobei beide gegenüber angeordnete schiefe Ebenen so im Eingriff stehen, dass bei einer linearen Bewegung des zweiten Hebels durch den Abzug die zweite schiefe Ebene und die erste schiefe Ebene zusammen mit dem ersten Hebel um wenigstens 5° aus der Längsachse der Vorrichtung ausschwenken, wobei durch die Schwenkbewegung des ersten Hebels das Verschlussteil im Ventilsitz gedreht wird. Dabei kann wiederum vorgesehen sein, dass ein elastisches Rückstellelement am ersten Hebel angeordnet ist, mit dem nach der, durch die lineare Bewegung der zweiten schiefen Ebene verursachten Schwenkbewegung des ersten Hebels, der erste Hebel in seine Ausgangsposition zurück gedrückt wird, und/oder dass ein elastisches Rückstellelement am zweiten Hebel angeordnet ist, das den zweiten Hebel nach erfolgter manueller linearer Bewegung zurück in die Ausgangsposition drückt.

Bevorzugt ist das Ventilelement als zylinderförmiges Drehventil, als Kugelventil oder als Klappenventil ausgebildet, wobei das Klappenventil besonders bevorzugt ist.

Mit einer Weiterentwicklung wird vorgeschlagen, dass in der Außenwand des Hohlzylinders, und zwar in der Hälfte, die in Richtung der Zweikomponentenkartusche ausgerichtet ist, und in der Wandung des Druckbehälters zumindest je eine durchgehende Entlüftungsöffnung vorgesehen ist, so dass der Druck aus dem Hohlzylinder entweicht, wenn der Kolben zwischen (axial zwischen) der Entlüftungsöffnungsöffnung und der Zweikomponentenkartusche angeordnet ist.

Hierdurch wird die Pasten-Applikationsvorrichtung nach dem Auspressen der Ausgangskomponenten druckfrei und kann anschließend gefahrlos recycelt oder entsorgt werden. Durch die Entlüftungsöffnungen ist der Raum zwischen der Zweikomponentenkartusche und den axial beweglichen Stößeln im Hohlzylinder mit der umgebenden Atmosphäre gasdurchlässig verbunden. Dabei kann vorgesehen sein, dass bei Erreichen der Endposition des Kolbens im Hohlzylinder das Druckgas in die Umgebung entweicht.

Bevorzugt ist die zumindest eine Entlüftungsöffnung des Hohlzylinders in der Zylindermantelwand des Hohlzylinders angeordnet. Ebenso bevorzugt ist die zumindest eine Entlüftungsöffnung des Druckbehälters in der Zylindermantelwand des Druckbehälters angeordnet. Es kann besonders bevorzugt auch vorgesehen sein, dass die Entlüftungsöffnungen im Hohlzylinder und im Druckbehälter einander überdeckend angeordnet sind. An der zumindest einen Entlüftungsöffnung des Druckbehälters kann ein manuell bedienbares Ventilelement zum Ablassen der Druckluft angeordnet sein.

Gemäß einer Weiterbildung kann vorgesehen sein, dass die Zweikomponentenkartusche, der Hohlzylinder und der Druckbehälter in einem Gehäuse angeordnet sind, wobei bevorzugt auch die Druckgaspatrone in dem Gehäuse angeordnet ist.

Hierdurch wird die Pasten-Applikationsvorrichtung nach außen abgeschlossen. Das Gehäuse besteht bevorzugt aus Kunststoff.

Bevorzugt kann auch vorgesehen sein, dass die Abstände zwischen dem Druckbehälter und der Außenwand der Zweikomponentenkartusche und zwischen dem Druckbehälter und der Außenwand des Hohlzylinders kleiner als 100 µm sind, bevorzugt kleiner als 50 µm sind.

Hierdurch kann der Druckbehälter der Pasten-Applikationsvorrichtung den Druck ohne größere Verformung des Hohlzylinders und der Zweikomponentenkartusche gut aufnehmen. Die Außenwandung der Zweikomponentenkartusche besteht vorzugsweise aus Kunststoff. Die Förderkolben können ebenfalls aus Kunststoff gefertigt werden, wobei bevorzugt zusätzlich umlaufende Dichtungen zum Abdichten der Förderkolben gegen die Innenräume an den Förderkolben angeordnet sind. An den Förderkolben können zudem Abstreiflippen angeordnet sein, mit denen der Inhalt der Innenräume vollständig oder zumindest zu wenigstens 99% aus den Innenräumen ausgetrieben werden kann.

Des Weiteren kann vorgesehen sein, dass die Pasten-Applikationsvorrichtung mit einer Hand zu halten ist und das Ventilelement mit der gleichen Hand zu bedienen ist. Hierdurch ist eine vereinfachte Anwendung der Pasten-Applikationsvorrichtung möglich. Die der vorliegenden Erfindung zugrundeliegenden Aufgaben werden auch gelöst durch ein Verfahren zum Mischen und Austreiben einer Paste mit einer erfindungsgemäßen Pasten-Applikationsvorrichtung mit den Schritten:
ein Druckgas wird aus der Druckgaspatrone durch den Anschluss des Hohlzylinders in den Hohlzylinder geleitet, wobei der Druckbehälter die auf den Wänden des Hohlzylinders lastende Kraft des Druckgases aufnimmt und der Kolben mit den Stößeln im Hohlzylinder durch das Druckgas in Richtung der Zweikomponentenkartusche vorangetrieben wird;
die vorangetriebenen Stößel des Kolbens treiben die Förderkolben in die zumindest zwei Innenräume der Zweikomponentenkartusche voran, wobei die Ausgangskomponenten aus den Innenräumen der Zweikomponentenkartusche durch die zumindest zwei Austragsöffnungen der Innenräume ausgetrieben werden,
wobei der Fluss der Ausgangskomponenten durch ein geschlossenes Ventilelement in Fließrichtung hinter den Austragsöffnungen gestoppt wird und durch eine manuellen Bedienung eines Bedienelements das Ventilelement geöffnet wird, so dass die Ausgangskomponenten und/oder deren Mischung durch das Ventilelement strömen und nach einem Mischen der Ausgangskomponenten die Mischung appliziert wird.

Es kann dabei vorgesehen sein, dass die beim Einleiten des Druckgases in den Hohlzylinder und die beim Vortreiben des Kolbens und der Förderkolben in der Zweikomponenten-Kartusche auftretenden Kräfte durch den Druckbehälter aufgenommen werden, insbesondere durch einen im wesentlichen zylindrischen Druckbehälter aufgenommen werden, der die Zweikomponentenkartusche und den Hohlzylinder umschließt, wobei der Druckbehälter besonders bevorzugt an der Zweikomponentenkartusche und dem Hohlzylinder anliegt.

Ferner kann vorgesehen sein, dass bei einer fehlenden manuellen Krafteinwirkung auf das Bedienelement das Ventilelement durch die Einwirkung einer Kraft eines Rückstellelements, insbesondere einer elastischen Feder, geschlossen wird.

Schließlich wird mit dem Verfahren auch vorgeschlagen, dass vor dem Einleiten des Druckgases ein Verschluss von der Pasten-Applikationsvorrichtung entfernt wird, der die Austragsöffnungen der Innenräume der Zweikomponentenkartusche verschließt, und ein Austragsrohr vor dem Austragsöffnungen der Innenräume befestigt wird, in dem das Ventilelement angeordnet ist. Dabei kann bevorzugt vorgesehen sein, dass die Druckgaspatrone nach dem Befestigen des Austragsrohrs geöffnet wird. Dabei wiederum kann besonders bevorzugt vorgesehen sein, dass mit dem Öffnen der Druckgaspatrone das Druckgas in den Hohlzylinder geleitet wird.

Der Erfindung liegt die überraschende Erkenntnis zugrunde, dass es mit Hilfe eines Druckbehälters und eines Ventilelements, das in Fließrichtung hinter den Kartuschen (den Innenräumen der Zweikomponentenkartusche) angeordnet ist und das mit viskosen Ausgangskomponenten gefüllt ist, gelingt, einen direkten Antrieb der Förderkolben mit dem Gasdruck aus einer Druckgaspatrone zu erzeugen, ohne dass der Druck des Gases zuvor reduziert werden müsste. Der Druckbehälter ist in der Lage, die großen auftretenden Kräfte aufzunehmen, ohne dass sich die innenliegenden Kunststoff-Formkörper (die Zweikomponentenkartusche und der Hohlzylinder) verformen oder zu stark verformen. Die innenliegenden Kunststoff-Formkörper sollten aus Kunststoff gefertigt sein, damit die Antriebselemente (der Kolben und die Förderkolben) gut in den Kunststoff-Formkörpern gleiten können und gleichzeitig abdichten. Das Ventilelement muss dazu ausreichend stabil konstruiert werden. Die Positionierung in einem relativ engen Kanal hinter den Innenräumen der Zweikomponentenkartusche hilft dabei, den Aufbau des Ventilelements ohne großen Konstruktionsaufwand stabil genug zu gestalten. Theoretisch kann das Ventilelement auch in Fließrichtung hinter einem statischen Mischer angeordnet werden, der die auf das Ventilelement wirkende Kraft dann weiter reduziert. Der Aufbau kann so relativ einfach und funktionell gestaltet werden, ohne dass aufwendige Bauteile zur Leitung und Beeinflussung des Druckgasflusses notwendig wären. Durch die Reduzierung der Anzahl der Bauteile wird auch die Anfälligkeit der Pasten-Applikationsvorrichtung für Fehlfunktionen oder Störungen erheblich reduziert.

Eigene Versuche mit Druckgas-getriebenen Pasten-Applikationsvorrichtungen zeigten, dass es entscheidend ist, dass die mit Druckgas beaufschlagten Bauteile gasdicht sind und sich nicht durch den Druck derart stark deformieren, dass die Funktion der Pasten-Applikationsvorrichtung eingeschränkt würde. In weiteren Versuchen wurde gefunden, dass es sehr schwierig ist, Zementkartuschen während des Austrags der Zementpasten, während des Vorschubs der Förderkolben durch Stößel, so zu lagern, dass sich die Zementkartuschen bei Innendrücken größer 20 bar nicht von den Stößeln und dem Stößelantrieb durch Verbiegung oder Verwindung entfernen. Eigene Versuche zeigten weiterhin, dass aus Aluminiumlegierungen gefertigte Zementkartuschen für Polymethylmethacrylat-Zementpasten ungeeignet sind, weil die Legierungen und die darin enthaltenden Legierungsbestandteile wie Kupfer und Mangan eine unerwünschte radikalische Polymerisationen währen der Lagerung der Zementpasten in der Zementkartusche beziehungsweise den Innenräumen bewirken. Nur in Zementkartuschen aus geeigneten Kunststoffen, wie Poly-acrylnitril-co-methylmethacrylat und Polybutylenterephthalat, sind Polymethylmethacrylat enthaltende Zementpasten ausreichend dauerhaft lagerstabil.

Im Folgenden wird ein erstes sehr allgemeines Ausführungsbeispiel der Erfindung beschrieben:
Eine beispielhafte Pasten-Applikationsvorrichtung ist zusammengesetzt aus
a) einem hohlzylinderförmiger ersten Druckbehälter mit einer Mindestdruckfestigkeit von 30 bar,
b) mindestens einer Kunststoffkartusche mit einem axial beweglichen Austragskolben, die im ersten Druckbehälter angeordnet ist,
c) einem Austragsrohr, das einen statischen Mischer und ein Ventilelement enthält und das formschlüssig mit der Kunststoffkartusche verbunden ist, wobei das Ventilelement außerhalb des ersten Druckbehälters angeordnet ist,
d) einen Kunststoffhohlzylinder mit einer offenen und einer geschlossenen Stirnseite, wobei an der Außenseite des Kunststoffhohlzylinders ein Hohlzylinder angeordnet ist, der mit dem Innenraum des Kunststoffhohlzylinders gasdurchlässig verbunden ist, und der an seiner Außenseite ein Außengewinde hat,
e) einen hohlzylinderförmiger zweiten Druckbehälter mit einer Mindestdruckfestigkeit von 30 bar, mit einer offenen Stirnseite und einer geschlossenen Stirnseite, wobei die geschlossene Stirnseite einen Durchbruch hat, wobei
f) der Kunststoffhohlzylinder so im zweiten Druckbehälter angeordnet ist, dass der Hohlzylinder durch den Durchbruch des zweiten Druckbehälters ragt,
g) der Spalt zwischen dem Kunststoffhohlzylinder und dem zweiten Druckbehälter kleiner 0,1 mm ist,
h) ein axial beweglicher Kolben (Antriebskolben) im Kunststoffhohlzylinder angeordnet ist, wobei der Kolben mindestens mit einem Stößel verbunden ist,
i) eine Öffnungsvorrichtung für eine Gaspatrone außerhalb des Druckbehälters angeordnet ist, die über ein Innengewinde mit dem Hohlzylinder verschraubt ist,
j) die beiden Druckbehälter und formschlüssig oder stoffschlüssig miteinander verbunden sind, und
k) einem geschlossenen Gehäuse, in dem der erste Druckbehälter, der zweite Druckbehälter, die Öffnungsvorrichtung der Gaspatrone und die Gaspatrone angeordnet sind.

Der Druckbehälter, beziehungsweise die Druckbehälter sind bevorzugt aus Metall und/oder aus Kunststoff und/oder aus Glasfaser-verstärktem Kunststoff gebildet. Besonders bevorzugt bestehen die Druckbehälter aus Metall. Aluminiumlegierungen, Stahl oder auch Zink sind dabei ganz besonders bevorzugt. Der Druckbehälter kann durch Fließpressen, Druckguss oder durch mechanische Bearbeitungsverfahren hergestellt sein. Besonders vorteilhaft ist die Herstellung von Druckbehältern aus Aluminiumlegierungen durch Fließpressen.

Erfindungsgemäß ist, dass der Hohlkörper mit dem Antriebskolben mindestens eine gasdurchlässige Öffnung besitzt, die mit mindestens einer gasdurchlässigen Öffnung des zweiten Druckbehälters gasdurchlässig verbunden ist, wobei die gasdurchlässige Öffnung des Hohlkörpers so angeordnet ist, dass bei Erreichen der Endposition des Antriebskolbens das Druckgas in die Umgebung entweicht. Dadurch wird die Vorrichtung drucklos nach dem vollständigen Austrag der Zementpasten und sichert sich dadurch selbst. Die drucklose Vorrichtung kann problemlos entsorgt werden.

Es ist erfindungsgemäß, dass das Ventilelement im Austragsrohr von außen zu betätigen ist, wobei das Ventilelement bevorzugt durch eine manuell ausgelöste Drehbewegung geöffnet und geschlossen wird, wobei besonders bevorzugt das Ventilelement als zylinderförmiges Drehventil, als Kugelventil oder als Klappenventil ausgebildet ist und wobei das Klappenventil ganz besonders bevorzugt ist.

Vorteilhaft ist es, wenn das Verschlussteil des Ventilelements mit einem Hebel verbunden ist, der an der Außenseite des Gehäuses parallel zur Längsachse angeordnet ist, wobei der Hebel zum Öffnen und Schließen des Ventilelements manuell bewegt wird, so dass sich das Verschlussteil im Ventilsitz dreht. Dadurch kann der medizinische Anwender problemlos den Austrag des gemischten Zementteigs regulieren.

In einer weiteren Ausgestaltung der Erfindung ist das Verschlussteil kraftschlüssig oder formschlüssig mit einer manuellen Abzugsvorrichtung verbunden, die parallel zur Längsachse der Vorrichtung angeordnet ist, wobei bei manueller linearer Bewegung der Abzugsvorrichtung parallel zur Längsachse die lineare Bewegung über ein Getriebe in eine zu mindestens partielle Drehbewegung des Verschlussteils überführt wird. Dadurch kann die Vorrichtung mit nur einer Hand gehalten und gesteuert werden. Ein Koppelgetriebe wird als Getriebe bevorzugt.

Besonders vorteilhaft ist es, wenn das Verschlussteil des Ventilelements mit einem ersten Hebel verbunden ist, wobei ein Hebelende des ersten Hebels als erste schiefe Ebene mit einem Winkel von 20° bis 45° ausgebildet ist, und dieser ersten schiefen Ebene gegenüber eine zweiten schiefen Ebene eines zweiten Hebels angeordnet ist, wobei der Hebel mit einem manuell zu betätigenden Abzug verbunden ist, wobei beide einander gegenüber angeordnete schiefe Ebenen so im Eingriff stehen, dass bei einer linearen Bewegung des zweiten Hebels durch den Abzug die zweite schiefe Ebene die erste schiefe Ebene zusammen mit dem ersten Hebel um wenigstens 5° aus der Längsachse der Vorrichtung ausschwenkt, wobei durch die Schwenkbewegung des ersten Hebels das Verschlussteil im Ventilsitz gedreht wird.

Weiterhin ist es vorteilhaft wenn ein elastisches Rückstellelement am ersten Hebel angeordnet ist, mit dem nach der durch die lineare Bewegung der zweiten schiefen Ebene verursachten Schwenkbewegung des ersten Hebels, der erste Hebel in seine Ausgangsposition zurück gedrückt wird.

Es ist außerdem vorteilhaft, wenn ein elastisches Rückstellelement am zweiten Hebel angeordnet ist, das den zweiten Hebel nach erfolgter manueller linearer Bewegung zurück in die Ausgangsposition drückt.

Im Folgenden werden weitere Ausführungsbeispiele der Erfindung anhand von acht schematisch dargestellten Figuren erläutert, ohne jedoch dabei die Erfindung zu beschränken. Dabei zeigt:
Figur 1: eine Querschnittansicht einer erfindungsgemäßen Pasten-Applikationsvorrichtung;
Figur 2: eine schematische Seitenansicht auf die Pasten-Applikationsvorrichtung nach Figur 1;
Figur 3: eine perspektivische Ansicht der Pasten-Applikationsvorrichtung nach den Figuren 1 und 2 mit geöffnetem Gehäuse;
Figur 4: eine schematische perspektivische Querschnittansicht der Pasten-Applikationsvorrichtung nach den Figuren 1 bis 3;
Figur 5: eine vergrößerte Teilansicht des Anschlusses für die Gaspatrone der Pasten-Applikationsvorrichtung nach Figur 4;
Figur 6: eine vergrößerte Teilansicht des Ventilelements für die Gaspatrone der Pasten-Applikationsvorrichtung nach Figur 4;
Figur 7: eine perspektivische Ansicht einer alternativen Pasten-Applikationsvorrichtung, mit einer anderen Bedieneinrichtung für das Ventilelement; und
Figur 8: eine perspektivische Ansicht der Pasten-Applikationsvorrichtung nach Figur 7 mit geöffnetem Gehäuse.

In den Figuren werden teilweise für gleiche oder gleichartige Bauteile die gleichen Bezugszeichen verwendet, auch wenn es sich um unterschiedliche Pasten-Applikationsvorrichtungen handelt.

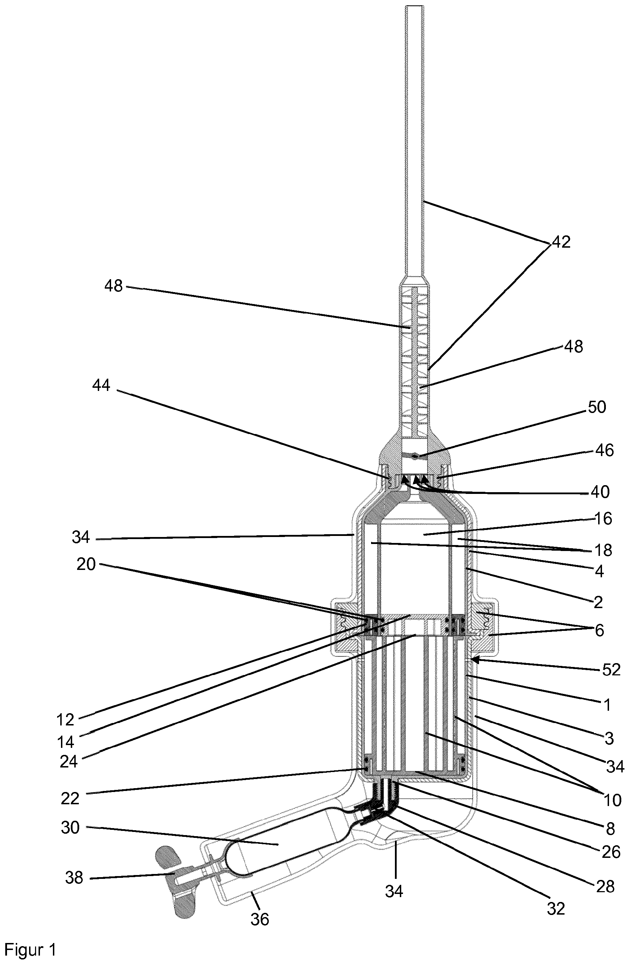

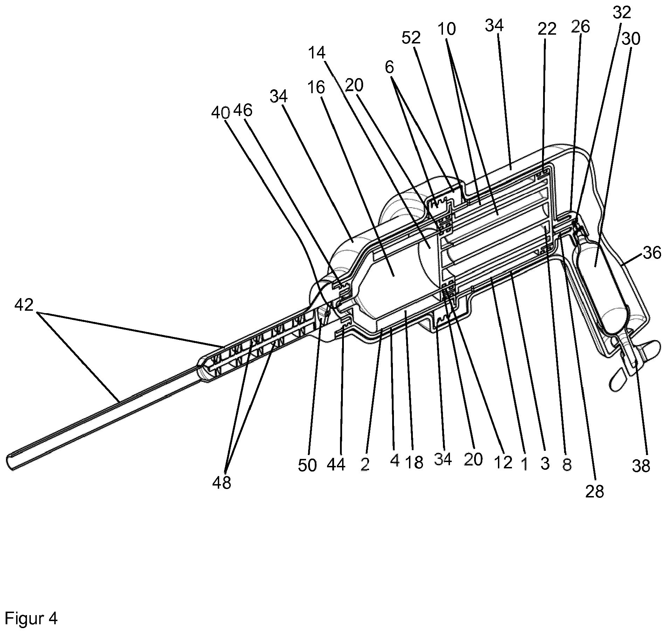

Die Figuren 1 bis 6 zeigen unterschiedliche Ansichten einer erfindungsgemäßen Druckgas-getriebenen Pasten-Applikationsvorrichtung beziehungsweise von Teilen davon. Die Pasten-Applikationsvorrichtung ist nach Art einer Pistole aufgebaut und kann in einer Hand gehalten und mit der anderen Hand bedient werden. Im Inneren der Pasten-Applikationsvorrichtung befindet sich ein Hohlzylinder 1 aus Kunststoff und eine benachbart angeordnete Zweikomponentenkartusche 2. Der Hohlzylinder 1 ist von einem ersten Teil eines Druckbehälters 3 umschlossen und die Zweikomponentenkartusche 2 ist von einem zweiten Teil eines Druckbehälters 4 umschlossen. Die beiden Teile des Druckbehälters 3, 4 bestehen aus einem metallischen Werkstoff, wie einer Aluminium-Legierung, Zink oder Stahl, und sind mit Hilfe einer zweiteiligen Überwurfmutter 6 formschlüssig an einer umlaufenden, nach außen gerichteten Falz umlaufend stoffschlüssig miteinander verbunden. Die Außenwände des Hohlzylinders 1 und der Zweikomponentenkartusche 2 liegen an den Innenwänden des Druckbehälters 3, 4 flächig an oder weisen einen Anstand von maximal 100 µm auf, damit der Druckbehälter 3, 4 Kräfte, die auf die Wandungen des Hohlzylinders 1 und der Zweikomponentenkartusche 2 wirken, aufnehmen kann, ohne dass eine für die Funktion der Pasten-Applikationsvorrichtung störende Verformung der Wandungen erfolgt. Der Hohlzylinder 1, die Zweikomponentenkartusche 2 und der Druckbehälter 3, 4 haben eine zylindrische Form beziehungsweise eine im Wesentlichen zylindrische Form.

Im Inneren des Hohlzylinders 1 ist ein Kolben 8 als Arbeitskolben vorgesehen, mit dem die im Druckgas gespeicherte Energie in eine lineare Bewegung entlang der Zylinderachse beziehungsweise in Längsrichtung der Pasten-Applikationsvorrichtung umgewandelt werden kann. An dem Kolben 8 sind zwei koaxial zueinander angeordnete Stößel 10 befestigt, die in Richtung der Zweikomponentenkartusche 2 ausgerichtet sind. Mit den Stößeln 10 können zwei Förderkolben 12, 14 angetrieben werden, die in koaxial zueinander angeordneten Innenräumen 16, 18 der Zweikomponentenkartusche 2 angeordnet sind. Die Förderkolben 12, 14 sind mit umlaufenden Dichtungen 20 gegen die Innenwände der Innenräume 16, 18 abgedichtet. Ebenso liegt der Kolben 8 umlaufend an der Innenwand des Hohlzylinders 1 an und ist dort mit umlaufenden Dichtungen 22 abgedichtet.

Zwischen dem Hohlzylinder 1 und den Stößeln 10 und der Zweikomponentenkartusche 2 ist eine Aluminium-Verbundfolie 24 auf die durch die Förderkolben 12, 14 geschlossenen Enden der Zweikomponentenkartusche 2 aufgeklebt. Die Aluminium-Verbundfolie 24 ist nur in Figur 1 eingezeichnet und in den anderen Figuren weggelassen. Hierdurch wird der Inhalt der Zweikomponentenkartusche 2 abgedichtet. Insbesondere wenn leicht flüchtige Bestandteile in den Ausgangskomponenten enthalten sind, die in den Innenräumen 16, 18 der Zweikomponentenkartusche 2 gelagert sind, wird ein entweichen dieser Bestandteile und damit eine Veränderung der Ausgangskomponenten bei der Lagerung vermieden. Beim Vortreiben der Förderkolben 12, 14 mit den Stößeln 10 des Kolbens 8 wird die Aluminium-Verbundfolie 24 einfach durchstoßen.

Das Druckgas mündet durch einen Anschluss 26 in den Hohlzylinder 1 zwischen der Rückseite des Kolbens 8 und der geschlossenen Seite des ersten Teils des Druckbehälters 3. Der Anschluss 26 ragt dazu durch die einzige Öffnung in der ansonsten geschlossenen Grundfläche des ersten Teils des Druckbehälters 3. Der Anschluss 26 ist einteilig mit dem Hohlzylinder 1 ausgeführt. Der Anschluss 26 weist ein Außengewinde auf, auf das ein Anschlussstück 28 aus Kunststoff geschraubt ist.

An das Anschlussstück 28 ist eine Druckgaspatrone 30 angeschlossen, die geöffnet werden kann, indem die Druckgaspatrone 30 auf einen Hohldorn 32 gedrückt wird. Dadurch öffnet sich die Druckgaspatrone 30 und das Druckgas kann durch die Leitungen in dem Anschlussstück 28 und den Anschluss 26 in den Hohlzylinder 1 strömen und den Kolben 8 in Richtung der Zweikomponentenkartusche 2 vortreiben. Die Druckgaspatrone 30 ist bevorzugt eine Flüssiggas-Patrone, in der besonders bevorzugt Kohlendioxid verdampft, um das Druckgas bereitzustellen.

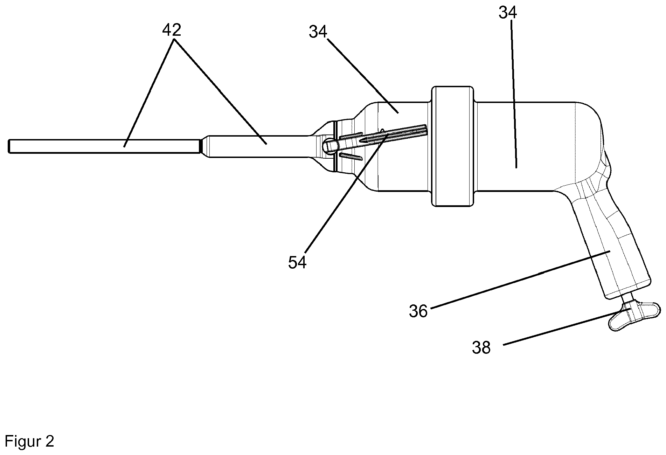

Der gesamte bisher genannte Aufbau beziehungsweise alle diese Teile (mit den Bezugszeichen 1 bis 32) sind innerhalb eines Gehäuses 34 aus Kunststoff angeordnet. Das Gehäuse 34 ist an der Rückseite (in Figur 1 unten und in den Figuren 2, 3 und 4 rechts) als Griff 36 beziehungsweise Pistolengriff 36 ausgebildet, mit dem die Pasten-Applikationsvorrichtung gehalten werden kann. An der Druckgaspatrone 20 ist bodenseitig eine Bedienvorrichtung 38 befestigt, die in einer Flügelschraube endet, mit denen die Druckgaspatrone 20 in das Anschlussstück 28 eingeschraubt und durch den Hohldorn 32 geöffnet werden kann. Die Pasten-Applikationsvorrichtung kann also manuell dadurch aktiviert werden beziehungsweise Startklar gemacht werden, dass die Bedienvorrichtung 38 bedient und damit die Druckgaspatrone 30 geöffnet wird.

An der Vorderseite des zylindrischen Teils der Zweikomponentenkartusche 2 läuft diese und die koaxial angeordneten Innenräume 16, 18 darin konisch zusammen und münden in Austragsöffnungen 40, durch die die Ausgangskomponenten aus den Innenräumen 16, 18 aus der Zweikomponentenkartusche 2 ausgetrieben werden, in Fließrichtung hinter den Austragsöffnungen 40 mischen sich dann die Ausgangskomponenten. Die Austragsöffnungen 40 sind im Lagerungszustand der Pasten-Applikationsvorrichtung zunächst durch einen schraubbaren Verschluss (nicht gezeigt) verschlossen. Im Anwendungszustand wird der Verschluss entfernt und dafür ein Austragsrohr 42 an der Vorderseite befestigt. Dazu ist an der Vorderseite ein Innengewinde 44 vorgesehen, das in einem Stutzen an der Zweikomponentenkartusche 2 ausgebildet ist. In dieses Innengewinde 44 ist ein Außengewinde 46 des Austragsrohrs 42 geschraubt. Der Verschluss (nicht gezeigt) weist ein analoges Außengewinde auf, das in das Innengewinde 44 der Zweikomponentenkartusche 2 geschraubt war beziehungsweise werden kann, um die Austragsöffnungen 40 zu verschließen.

Im Inneren des Austragsrohrs 42 ist ein statischer Mischer 48 vorgesehen, mit dem die Ausgangskomponenten durchmischt werden, wenn diese durch das Austragsrohr 42 strömen. Der Durchfluss durch das Austragsrohr 42 kann mit einem manuell bedienbaren Klappventil 50 gesteuert werden, das im Kanal des Austragsrohrs 42 drehbar gelagert ist. Wenn das Klappventil 50 geschlossen ist, können die Ausgangskomponenten nicht weiter aus der Zweikomponentenkartusche 2 ausgetrieben werden und demzufolge die Förderkolben 12, 14 und der Kolben 8 nicht weiter vorangetrieben werden. Dadurch, dass nicht der Gasstrom unterbrochen wird, wie bei anderen aus dem Stand der Technik bekannten Applikationsvorrichtungen, kann es nicht zu einer weiteren Expansion des bereits eingeleiteten Gases kommen und damit zu einem unerwünschten Nachlaufen der Pasten-Mischung. Die Anordnung des Klappventils 50 in dem Kanal des Austragsrohrs 42 ist auch insofern geschickt, da aufgrund des geringen Querschnitts die Belastung der Lager des Klappventils 50 begrenzt ist.

In dem Hohlzylinder 1 und dem ersten Teil des Druckbehälters 3 sind übereinander liegend mehrere Entlüftungsöffnungen 52 vorgesehen. Die Entlüftungsöffnungen 52 sind in einer Höhe des Hohlzylinders 1 angeordnet, die zumindest und bevorzugt in etwa der Höhe des Kolbens 8 entspricht, so dass wenn der Kolben 8 bis zum Anschlag in Richtung der Zweikomponentenkartusche 2 vorgetrieben ist, der Bereich zwischen dem Kolben 8 und der Rückseite des Hohlzylinders 1 zur Umgebung durch die Entlüftungsöffnungen 52 geöffnet wird, und der verbleibende Druck in die Umgebung entweichen kann. Hierdurch wird sichergestellt, dass die verbrauchte Pasten-Applikationsvorrichtung beim Recyceln oder bei der Entsorgung nicht mehr unter Druck steht und daher gefahrlos weiterverarbeitet werden kann.

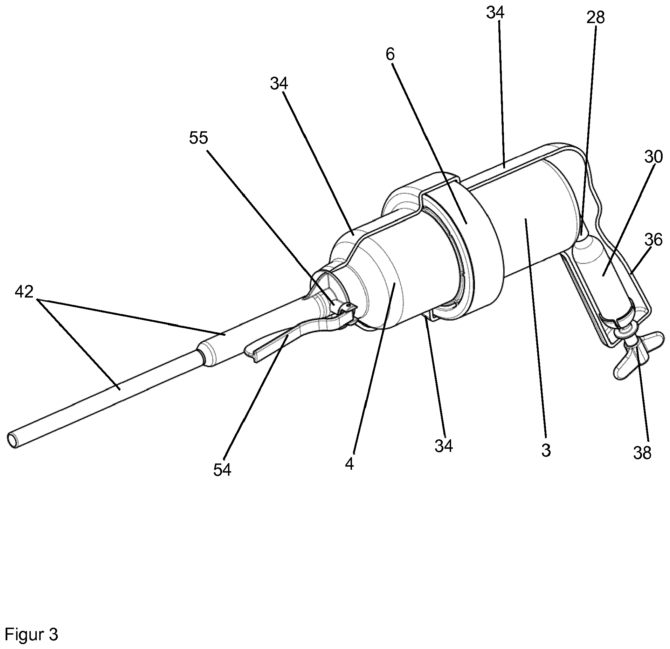

Das Klappventil 50 ist von außerhalb der Pasten-Applikationsvorrichtung bedienbar, in dem ein Bedienelement 54 (gezeigt in Figur 2 und 3) in Form eines Hebels 54 mit dem Klappventil 50 verbunden ist, so dass sich das Klappventil 50 durch den Hebel 54 im Austragsrohr 42 drehen lässt und damit der Fluss der Ausgangskomponenten und der Mischung steuern lässt. Dazu ist die Achse (in den Figuren 2 und 3 nicht zu erkennen) des Klappventils 50 in einem Lager 55 beziehungsweise einer Hülse 55 gelagert, durch die die Achse hindurchgeführt ist, so dass die Stellung des Klappventils 50 durch Drehen der Achse mit Hilfe des Hebels 54 einstellbar ist.

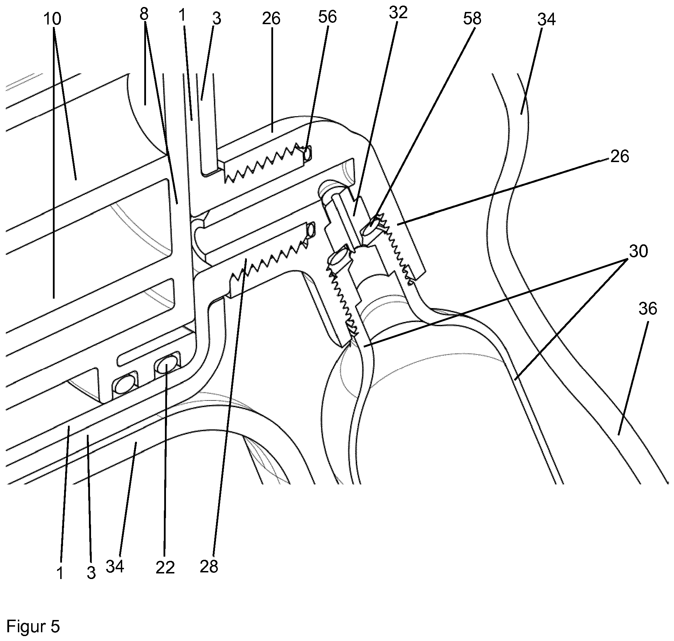

Wie in der vergrößerten Teilansicht nach Figur 5 dargestellt und gut zu erkennen ist, ist auf dem Anschluss 26 ein Außengewinde vorgesehen, auf das das Anschlussstück 28 mit einem Innengewinde aufgeschraubt ist. Um die beiden Teile 26, 28 druckdicht gegeneinander abzudichten, ist eine Dichtung 56 vorgesehen. Analog ist zwischen dem Anschlussstück 28 und der Druckgaspatrone 30 beziehungsweise zwischen dem Hohldorn 32 und der Druckgaspatrone 30 eine Dichtung 58 vorgesehen.

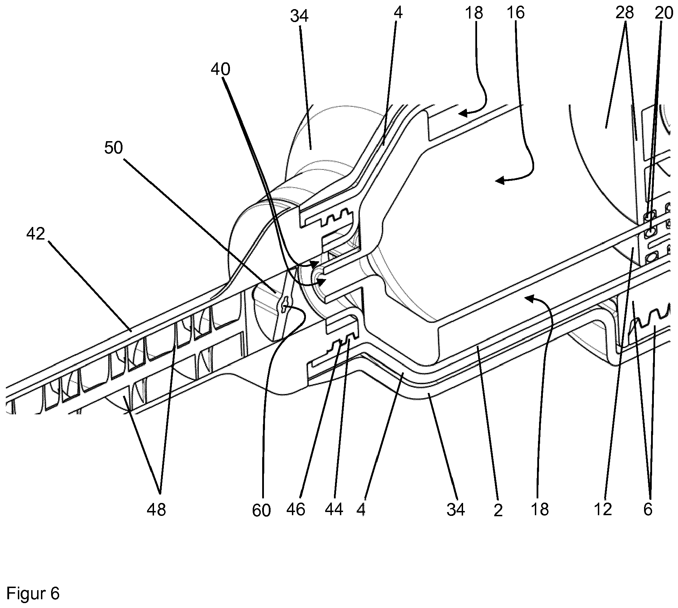

In der vergrößerten Teilansicht nach Figur 6 ist zu erkennen, dass das Klappenventil 50 eine Stahlachse 60 aufweist, um die das Klappenventil 50 in dem Austragsrohr 42 mit Hilfe des Bedienelements 54 drehbar ist. Die Stahlachse 60 sorgt für die nötige Stabilität des Klappenventils 50. Die Stahlachse 60 darf sich nämlich unter Einwirkung des Drucks aus der Druckgaspatrone 30 vermittelt durch die Ausgangskomponenten nicht derart plastisch Verformen, dass die Funktion des Klappenventils 50, beispielsweise die Beweglichkeit des Klappenventils 50, nicht mehr gewährleistet wäre. Durch Drehen des Klappenventils 50 um die Stahlachse 60 kann der Volumenfluss durch das Austragsrohr 42 gesteuert werden, während der Gasdruck vermittelt durch den Kolben 8 und die Förderkolben 12, 14 den Inhalt aus der Zweikomponentenkartusche 2 austreibt, das heißt, die Ausgangskomponenten aus der Zweikomponentenkartusche 2 austreibt.

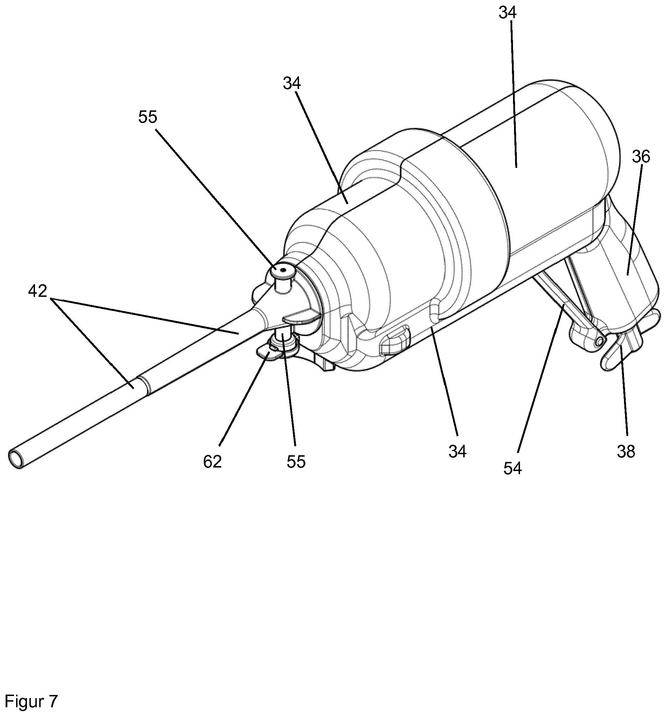

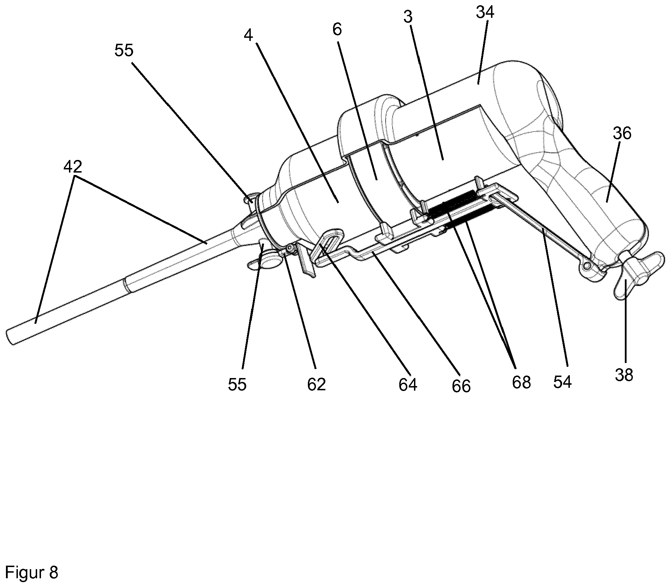

Figur 7 zeigt eine perspektivische Ansicht einer alternativen Pasten-Applikationsvorrichtung, mit einer anderen Bedieneinrichtung für das Ventilelement (in den Figuren 7 und 8 nicht zu sehen) und Figur 8 zeigt eine perspektivische Ansicht der Pasten-Applikationsvorrichtung nach Figur 7 mit geöffnetem Gehäuse 34. Der Aufbau dieser Pasten-Applikationsvorrichtung gleicht der nach den Figuren 1 bis 6, lediglich die Lagerung des Ventilelements und die Funktionsweise der Bedieneinrichtung ist eine andere. Das Ventilelement wird bei dieser Ausführung ebenfalls durch ein Klappenventil realisiert, wobei die Drehachse (aus Stahl) im Vergleich zur Anordnung nach den Figuren 1 bis 6 um 90° zur Zylindersymmetrieachse der Zweikomponentenkartusche gedreht ist. Die Drehachse ist in zwei hülsenförmigen Lagern 55 gelagert, von denen eine eine durchgehend Durchführung für die Drehachse aufweist, wobei die Drehachse an einer Stange 62 befestigt ist, mit der die Drehachse von außen gedreht werden kann.

Die Stange 62 ist mit einer Schiene 64 verbunden, die über eine Achse drehbar mit dem Gehäuse 34 verbunden ist. In der Schiene 64 läuft ein Zylinder, der an einer zweiten Stange 66 befestigt ist. Die zweite Stange 66 ist über ein Gelenk mit einer Bedienvorrichtung 54 verbunden, die kippbar am Pistolengriff 36 der Pasten-Applikationsvorrichtung befestigt ist. Zwei Zugfedern 68 sind als Rückstellelemente an der zweiten Stange 64 und an einer Halterung am Gehäuse 34 befestigt, mit denen die Bedienvorrichtung 54 beziehungsweise der Abzug 54 in die Ausgangsstellung rücküberführt wird, wenn keine Kraft mehr auf die Bedienvorrichtung 54 ausgeübt wird.

Die Bedieneinrichtung für das drehbare Klappenventil umfasst bei dieser Ausführung die Bedienvorrichtung 54, die Stangen 62, 66, die Schiene 66, die Federn 68 und alle Befestigungsmittel, die der Lagerung dieser Teile am Gehäuse 34 dienen.

Durch diesen Aufbau gelingt es, dass die gesamte Pasten-Applikationsvorrichtung mit einer Hand gehalten und auch bedient werden kann. Selbstverständlich sind auch andere Konfigurationen denkbar, mit denen eine solche einhändige Bedienbarkeit ermöglicht werden kann.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Hohlzylinder
- 2: Zweikomponentenkartusche
- 3: Druckbehälter, erster Teil
- 4: Druckbehälter, zweiter Teil
- 6: Überwurfmutter
- 8: Kolben
- 10: Stößel
- 12: Förderkolben
- 14: Förderkolben
- 16: Innerer Innenraum
- 18: Äußerer Innenraum
- 20: Dichtung
- 22: Dichtung
- 24: Aluminium-Verbundfolie
- 26: Anschluss für Druckgas
- 28: Anschlussstück
- 30: Druckgaspatrone
- 32: Stechdorn / Hohldorn
- 34: Gehäuse
- 36: Griff
- 38: Bedienvorrichtung
- 40: Austragsöffnung
- 42: Austragsrohr
- 44: Innengewinde
- 46: Außengewinde
- 48: Statischer Mischer
- 50: Drehbares Klappenventil
- 52: Entlüftungsöffnung
- 54: Bedienelement
- 55: Lager / Hülse
- 56: Dichtung
- 58: Dichtung
- 60: Stahlachse
- 62: Stange
- 64: Schiene
- 66: Stange
- 68: Zugfeder

## Patentansprüche

1. Pasten-Applikationsvorrichtung zum Lagern zweier Ausgangskomponenten, zum Mischen der Ausgangskomponenten zu einer Paste und zum Applizieren der Paste aufweisend
eine Zweikomponentenkartusche (2) aufweisend zwei zylindrische Innenräume (16, 18), zwei axial in den Innenräumen (16, 18) verschiebbare Förderkolben (12, 14), die die Innenräume (16, 18) auf einer ersten Seite der Zweikomponentenkartusche (2) begrenzen, und zumindest zwei Austragsöffnungen (40), durch die die Innenräume (16, 18) an einer der ersten Seite gegenüberliegenden zweiten Seite der Zweikomponentenkartusche (2) geöffnet sind, **gekennzeichnet durch**
einen Hohlzylinder (1) aus einem Kunststoff mit einer offenen Stirnseite und einer bereichsweise geschlossenen Stirnseite, in dem sich ein axial beweglicher Kolben (8) mit zwei daran befestigten Stößeln (10) befindet, wobei die Stößel (10) in Richtung der offenen Stirnseite ausgerichtet sind, wobei der Hohlzylinder (1) mit der offenen Stirnseite axial an der ersten Seite der Zweikomponentenkartusche (2) anliegend angeordnet ist und wobei der Kolben (8) gasdicht mit den Innenwänden des Hohlzylinders (1) abschließt,
wobei die Zweikomponentenkartusche (2) und der Hohlzylinder (1) in einem Druckbehälter (3, 4) aus Metall, einem hochfesten Kunststoff oder einem faserverstärkten Kunststoff angeordnet sind, wobei der Druckbehälter (3, 4) an dem Hohlzylinder (1) anliegt oder einen Abstand von höchstens 0,1 mm zum Hohlzylinder (1) aufweist,
wobei der Hohlzylinder (1) an der bereichsweise geschlossenen Stirnseite einen Anschluss (26) für eine Druckgaspatrone (30) aufweist, der sich durch eine Öffnung im Druckbehälter (3, 4) erstreckt und
wobei in Fließrichtung hinter den Austragsöffnungen (40) ein manuell bedienbares Ventilelement (50) befestigt ist oder ein Austragsrohr (42) mit einem manuell bedienbaren Ventilelement (50) zu befestigen ist, wobei mit dem hinter den Austragsöffnungen (40) befestigten Ventilelement (50) der Volumenfluss der Ausgangskomponenten durch die Austragsöffnungen (40) zu regulieren ist.

2. Pasten-Applikationsvorrichtung nach Anspruch 1, **dadurch gekennzeichnet, dass**
das Ventilelement (50) in einer Leitung angeordnet ist, die durch ein Austragsrohr (42) gebildet ist, wobei bevorzugt im Austragsrohr (42) zusätzlich ein statischer Mischer (48) vorgesehen ist, mit dem die Ausgangskomponenten beim Durchströmen durch den Mischer (48) durchmischbar sind.

3. Pasten-Applikationsvorrichtung nach Anspruch 1 oder 2, **dadurch gekennzeichnet, dass**
der Druckbehälter (3, 4) zweiteilig aufgebaut ist, wobei die beiden Teile (3, 4) durch Vernietung, durch Verschraubung und/oder durch eine Überwurfmutter (6) kraftschlüssig verbunden sind, wobei vorzugsweise ein erster Teil (4) der beiden Teile des Druckbehälters (3, 4) die Zweikomponentenkartusche (2) beinhaltet und der zweite (3) der beiden Teile des Druckbehälters (3, 4) den Hohlzylinder (1).

4. Pasten-Applikationsvorrichtung nach einem der vorangehenden Ansprüche,
**dadurch gekennzeichnet, dass**
die Zweikomponentenkartusche (2) zumindest bereichsweise eine Koaxialkartusche ist, wobei in der Koaxialkartusche einer der Innenräume (16) zylindrisch und innenliegend ist und der andere Innenraum (18) zylindrisch ist und den innenliegenden Innenraum (16) koaxial umschließt.

5. Pasten-Applikationsvorrichtung nach einem der vorangehenden Ansprüche,
**dadurch gekennzeichnet, dass**
die Pasten-Applikationsvorrichtung einen Verschluss zum Verschließen der Austragsöffnungen (40) aufweist, der mit einem Befestigungsmittel des Verschlusses, insbesondere mit einem Gewinde des Verschlusses, an einem Gegenbefestigungsmittel (44) im Bereich der Austragsöffnungen (40), insbesondere an einem Gegengewinde (44) im Bereich der Austragsöffnungen (40), zu befestigen ist, wobei vorzugsweise an dem Gegenbefestigungsmittel (44) das Austragsrohr (42) zu befestigen ist, das zu diesem Zweck ebenfalls ein dementsprechendes Befestigungsmittel (46) aufweist.

6. Pasten-Applikationsvorrichtung nach Anspruch 5, **dadurch gekennzeichnet, dass**
der Verschluss ein Schlüssel (38) zum Öffnen der Druckgaspatrone (30) und/oder zum Verbinden der Druckgaspatrone (30) mit dem Anschluss (26) des Hohlzylinders (1) ist, wobei vorzugsweise der Verschluss als Schlüssel (38) bodenseitig auf die Druckgaspatrone (30) zu stecken ist und damit die Druckgaspatrone (30) zum Öffnen der Druckgaspatrone (30) und/oder Verbinden der Druckgaspatrone (30) mit dem Anschluss (26) des Hohlzylinders (1) bewegbar, insbesondere drehbar und/oder in Längsrichtung verschiebbar ist.

7. Pasten-Applikationsvorrichtung nach einem der vorangehenden Ansprüche,
**dadurch gekennzeichnet, dass**
an dem Anschluss (26) des Hohlzylinders (1) ein Druckgaspatronenbefestigungsmittel (28) vorgesehen ist, mit dem die Druckgaspatrone (30) an dem Anschluss (26) druckdicht zu befestigen ist, und/oder an dem Anschluss (26) des Hohlzylinders (1) ein Stechdorn (32) zum Öffnen der Druckgaspatrone (30) vorgesehen ist.

8. Pasten-Applikationsvorrichtung nach einem der vorangehenden Ansprüche,
**dadurch gekennzeichnet, dass**
zwischen dem Hohlzylinder (1) und der Zweikomponentenkartusche (2) eine Schutzfolie (24) angeordnet ist, die die Innenräume (16, 18) der Zweikomponentenkartusche (2) auf der ersten Seite verschließen, wobei die Schutzfolie (24) vorzugsweise auf die Zweikomponentenkartusche (2) aufgeklebt, angeschweißt oder angehaftet ist.

9. Pasten-Applikationsvorrichtung nach einem der vorangehenden Ansprüche,
**dadurch gekennzeichnet, dass**
der Druckbehälter (3, 4) aus Aluminium, Zink, einer Aluminiumlegierung oder Stahl besteht.

10. Pasten-Applikationsvorrichtung nach einem der vorangehenden Ansprüche,
**dadurch gekennzeichnet, dass**
das manuell bedienbare Ventilelement (50) über ein Bedienelement (54) von außen bedienbar ist, vorzugsweise über einen Hebel (54) am Austragsrohr (42) oder einen Abzug (54) an einem Griff (36) der Pasten-Applikationsvorrichtung bedienbar ist.

11. Pasten-Applikationsvorrichtung nach einem der vorangehenden Ansprüche,
**dadurch gekennzeichnet, dass**
in der Außenwand des Hohlzylinders (1), und zwar in der Hälfte, die in Richtung der Zweikomponentenkartusche (2) ausgerichtet ist, und in der Wandung des Druckbehälters (3, 4) zumindest je eine durchgehende Entlüftungsöffnung (52) vorgesehen ist, so dass der Druck aus dem Hohlzylinder (1) entweicht, wenn der Kolben (8) zwischen der Entlüftungsöffnungsöffnung (52) und der Zweikomponentenkartusche (2) angeordnet ist.

12. Pasten-Applikationsvorrichtung nach einem der vorangehenden Ansprüche,
**dadurch gekennzeichnet, dass**
die Zweikomponentenkartusche (2), der Hohlzylinder (1) und der Druckbehälter (3, 4) in einem Gehäuse (34) angeordnet sind, wobei bevorzugt auch die Druckgaspatrone (30) in dem Gehäuse (34) angeordnet ist.

13. Pasten-Applikationsvorrichtung nach einem der vorangehenden Ansprüche,
**dadurch gekennzeichnet, dass**
die Abstände zwischen dem Druckbehälter (3, 4) und der Außenwand der Zweikomponentenkartusche (2) und zwischen dem Druckbehälter (3, 4) und der Außenwand des Hohlzylinders (1) kleiner als 100 µm sind, bevorzugt kleiner als 50 µm sind.

14. Pasten-Applikationsvorrichtung nach einem der vorangehenden Ansprüche,
**dadurch gekennzeichnet, dass**
die Pasten-Applikationsvorrichtung mit einer Hand zu halten ist und das Ventilelement (50) mit der gleichen Hand zu bedienen ist.

15. Verfahren zum Mischen und Austreiben einer Paste mit einer Pasten-Applikationsvorrichtung nach einem der Ansprüche 1 bis 14, **gekennzeichnet durch** die Schritte:
ein Druckgas wird aus der Druckgaspatrone (30) durch den Anschluss (26) des Hohlzylinders (1) in den Hohlzylinder (1) geleitet, wobei der Druckbehälter (3, 4) die auf den Wänden des Hohlzylinders (1) lastende Kraft des Druckgases aufnimmt und der Kolben (8) mit den Stößeln (10) im Hohlzylinder (1) durch das Druckgas in Richtung der Zweikomponentenkartusche (2) vorangetrieben wird;
die vorangetriebenen Stößel (10) des Kolbens (8) treiben die Förderkolben (12, 14) in die zumindest zwei Innenräume (16, 18) der Zweikomponentenkartusche (2) voran, wobei die Ausgangskomponenten aus den Innenräumen (16, 18) der Zweikomponentenkartusche (2) durch die zumindest zwei Austragsöffnungen (40) der Innenräume (16, 18) ausgetrieben werden,
wobei der Fluss der Ausgangskomponenten durch ein geschlossenes Ventilelement (50) in Fließrichtung hinter den Austragsöffnungen (40) gestoppt wird und durch eine manuellen Bedienung eines Bedienelements (54) das Ventilelement (50) geöffnet wird, so dass die Ausgangskomponenten und/oder deren Mischung durch das Ventilelement (50) strömen und nach einem Mischen der Ausgangskomponenten die Mischung appliziert wird.

16. Verfahren nach A, **dadurch gekennzeichnet, dass**
bei einer fehlenden manuellen Krafteinwirkung auf das Bedienelement (54) das Ventilelement (50) durch die Einwirkung einer Kraft eines Rückstellelements (68), insbesondere einer elastischen Feder (68), geschlossen wird.

17. Verfahren nach einem der Ansprüche 15 oder 16, **dadurch gekennzeichnet, dass**
vor dem Einleiten des Druckgases ein Verschluss von der Pasten-Applikationsvorrichtung entfernt wird, der die Austragsöffnungen (40) der Innenräume (16, 18) der Zweikomponentenkartusche (2) verschließt, und ein Austragsrohr (42) vor dem Austragsöffnungen (40) der Innenräume (16, 18) befestigt wird, in dem das Ventilelement (50) angeordnet ist, wobei bevorzugt die Druckgaspatrone (30) nach dem Befestigen des Austragsrohrs (42) geöffnet wird und besonders bevorzugt mit dem Öffnen der Druckgaspatrone (30) das Druckgas in den Hohlzylinder (1) geleitet wird.

## Claims

1. A paste application device for storing two starting components, for mixing the starting components to form a paste and for applying the paste, having
a two-component cartridge (2) having two cylindrical internal spaces (16, 18), two delivery pistons (12, 14), which can be shifted axially in the internal spaces (16, 18) and which limit the internal spaces (16, 18) on a first side of the two-component cartridge (2), and at least two discharge openings (40), by means of which the internal spaces (16, 18) are open on a second side of the two-component cartridge (2), which is located opposite the first side,
**characterised by**
a hollow cylinder (1) made of a plastic material comprising an open front face and a front face which is partially closed and in which an axially movable piston (8) is located comprising two plungers (10) attached thereto, wherein the plungers (10) are oriented in the direction of the open front face, wherein the open front face of the hollow cylinder (1) is arranged axially adjacent to the first side of the two-component cartridge (2), and wherein the piston (8) terminates in a gas-tight manner with the internal walls of the hollow cylinder (1).
wherein the two-component cartridge (2) and the hollow cylinder (1) are arranged in a pressure vessel (3, 4) made of metal, a high-strength plastic material or a fibre-reinforced plastic material, wherein the pressure vessel (3, 4) abuts on the hollow cylinder (1) or has a distance of maximally 0.1 mm from the hollow cylinder (1),
wherein, on the partially closed front face, the hollow cylinder (1) has a connector (26) for a compressed gas cartridge (30), which extends through an opening in the pressure vessel (3, 4), and
wherein a manually operable valve element (50) is fastened, or a discharge tube (42) comprising a manually operable valve element (50) is to be fastened in the flow direction downstream from the discharge openings (40), wherein the volume flow of the starting components through the discharge openings (40) can be regulated by means of the valve element (50), which is fastened downstream from the discharge openings (40).

2. The paste application device according to claim 1, **characterised in that** the valve element (50) is arranged in a conduit, which is formed by a discharge tube (42), wherein a static mixer is additionally provided, preferably in the discharge tube (42), by means of which the starting components can be mixed while flowing through the mixer (48).

3. The paste application device according to claim 1 or 2, **characterised in that** the pressure vessel (3, 4) is constructed in two parts, wherein the two parts (3, 4) are connected in a force-locking manner by means of riveting, by means of a screw connection and/or by means of a cap nut (6), wherein preferably a first part (4) of the two parts of the pressure vessel (3, 4) contains the two-component cartridge (2), and the second (3) one of the two parts of the pressure vessel (3, 4) contains the hollow cylinder (1).

4. The paste application device according to any one of the preceding claims,
**characterised in that**
the two-component cartridge (2) is at least partially a coaxial cartridge, wherein one of the internal spaces (16) in the coaxial cartridge is cylindrical and located on the inside, and the other internal space (18) is cylindrical and coaxially surrounds the inner internal space (16).

5. The paste application device according to any one of the preceding claims,
**characterised in that**
the paste application device has a closure for closing the discharge openings (40), which is to be fastened to a counter fastening means (44) in the area of the discharge openings (40), in particular to a counter thread (44) in the area of the discharge openings (40), by means of a fastening means of the closure, in particular by means of a thread of the closure, wherein the discharge tube (42), which, for this purpose, also has a corresponding fastening means (46), is to be fastened preferably to the counter fastening means (44).

6. The paste application device according to claim 5, **characterised in that** the closure is a key (38) for opening the compressed gas cartridge (30) and/or for connecting the compressed gas cartridge (30) to the connector (26) of the hollow cylinder (1), wherein the closure can preferably be inserted as a key (38) into the compressed gas cartridge (30) on the bottom side, and the compressed gas cartridge (30) can thus be moved, in particularly rotated and/or shifted in longitudinal direction, for opening the compressed gas cartridge (30) and/or for connecting the compressed gas cartridge (30) to the connector (26) of the hollow cylinder (1).

7. The paste application device according to any one of the preceding claims,
**characterised in that**
a compressed gas cartridge fastening means (28) is provided on the connector (26) of the hollow cylinder (1), by means of which the compressed gas cartridge (30) can be fastened to the connector (26) in a pressure-tight manner, and/or a piercing mandrel (32) for opening the compressed gas cartridge (30) is provided on the connector (26) of the hollow cylinder (1).

8. The paste application device according to any one of the preceding claims,
**characterised in that**
a protective film (24), which closes the internal spaces (16, 18) of the two-component cartridge (2) on the first side, is arranged between the hollow cylinder (1) and the two-component cartridge (2), wherein the protective film (24) is preferably pasted, welded or adhered to the two-component cartridge (2).

9. The paste application device according to any one of the preceding claims,
**characterised in that**
the pressure vessel (3, 4) consists of aluminium, zinc, an aluminium alloy or steel.

10. The paste application device according to any one of the preceding claims,
**characterised in that**
the manually operable valve element (50) can be operated from outside via an operating element (54), preferably via a lever (54) on the discharge tube (42) or a trigger (54) on a handle (36) of the paste application device.

11. The paste application device according to any one of the preceding claims,
**characterised in that**
at least one continuous ventilation opening (52) is in each case provided in the external wall of the hollow cylinder (1), namely **in that** half which is oriented in the direction of the two-component cartridge (2), and in the wall of the pressure vessel (3, 4), so that the pressure escapes from the hollow cylinder (1) when the piston (8) is arranged between the ventilation opening (52) and the two-component cartridge (2).

12. The paste application device according to any one of the preceding claims,
**characterised in that**
the two-component cartridge (2), the hollow cylinder (1), and the pressure vessel (3, 4) are arranged in a housing (34), wherein the compressed gas cartridge (30) is preferably also arranged in the housing (34).

13. The paste application device according to any one of the preceding claims,
**characterised in that**
the distances between the pressure vessel (3, 4) and the external wall of the two-component cartridge (2) and between the pressure vessel (3, 4) and the external wall of the hollow cylinder (1) are smaller than 100 µm, preferably smaller than 50 µm.

14. The paste application device according to any one of the preceding claims,
**characterised in that**
the paste application device can be held with one hand and the valve element (50) can be operated with the same hand.

15. A method for mixing and discharging a paste by means of a paste application device according to any one of claims 1 to 14, **characterised by** the steps:
a compressed gas is guided from the compressed gas cartridge (30) through the connector (26) of the hollow cylinder (1) into the hollow cylinder (1),
wherein the pressure vessel (3, 4) absorbs the force of the compressed gas acting on the walls of the hollow cylinder (1), and the piston (8) comprising the plungers (10) in the hollow cylinder (1) is advanced in the direction of the two-component cartridge (2) by the compressed gas;
the advanced plungers (10) of the piston (8) advance the delivery pistons (12, 14) into the at least two internal spaces (16, 18) of the two-component cartridge (2), wherein the starting components are expelled from the internal spaces (16, 18) of the two-component cartridge (2) through the at least two discharge openings (40) of the internal spaces (16, 18),
wherein the flow of the starting components is stopped by means of a closed valve element (50) in the flow direction downstream from the discharge openings (40), and the valve element (50) is opened by means of a manual operation of an operating element (54), so that the starting components and/or the mixture thereof flow through the valve element (50) and the mixture is applied after mixing the starting components.

16. The method according to A, **characterised in that**,
in the absence of a manual force acting on the operating element (54), the valve element (50) is closed under the impact of a force of a restoring element (68), in particular of an elastic spring (68).

17. The method according to any one of claims 15 or 16, **characterised in that**, prior to introducing the compressed gas, a closure is removed from the paste application device, which closes the discharge openings (40) of the internal spaces (16, 18) of the two-component cartridge (2), and a discharge tube (42) is fastened upstream of the discharge openings (40) of the internal spaces (16, 18), in which the valve element (50) is arranged, wherein the compressed gas cartridge (30) is preferably opened after fastening the discharge tube (42) and the compressed gas is guided into the hollow cylinder (1) particularly preferably while the compressed gas cartridge (30) is being opened.

## Revendications

1. Dispositif d'application de pâte pour le stockage de deux composants de départ, pour le mélange des composants de départ en une pâte et pour l'application de la pâte présentant
une cartouche à deux composants (2) présentant deux espaces intérieurs cylindriques (16, 18), deux pistons d'alimentation (12, 14) déplaçables en sens axial dans les espaces intérieurs (16, 18) qui limitent les espaces intérieurs (16, 18) sur une première face de la cartouche à deux composants (2) et au moins deux orifices de sortie (40), par lesquels les espaces intérieurs (16, 18) sont ouverts sur une deuxième face de la cartouche à deux composants (2), opposée à la première face, **caractérisé par le fait**
**qu'**un cylindre creux (1) en matière synthétique avec une face frontale ouverte et une face frontale localement fermée, dans lequel se trouve un piston (8) mobile en sens axial avec deux coulisseaux (10) fixés sur celui-ci, les coulisseaux (10) étant orientés vers la face frontale ouverte, le cylindre creux (1) étant disposé de manière adjacente avec la face frontale ouverte en sens axial sur la première face de la cartouche à deux composants (2) et le piston (8) assurant une fermeture étanche au gaz avec les parois intérieures du cylindre creux (1),
la cartouche à deux composants (2) et le cylindre creux (1) étant disposés dans un réservoir sous pression (3, 4) en métal, matière synthétique hautement résistante ou matière synthétique renforcée par des fibres, le réservoir sous pression (3, 4) adhérant sur le cylindre creux (1) ou présentant une distance maximum de 0,1 mm par rapport au cylindre creux (1),
le cylindre creux (1) présentant, sur la face frontale localement fermée, un raccord (26) pour une cartouche de gaz comprimé (30) qui s'étend par un orifice dans le réservoir sous pression (3, 4) et
un élément de vanne (50) commandé manuellement étant fixé dans le sens de l'écoulement derrière les orifices de sortie (40) ou un tuyau de vidange (42) étant à fixer avec un élément de vanne (50) commandé manuellement, le débit volumétrique des composants de départ étant à réguler par les orifices de sortie (40) avec l'élément de vanne (50) fixé derrière les orifices de sortie (40).

2. Dispositif d'application de pâte conformément à la revendication n°1,
**caractérisé par le fait que**
l'élément de vanne (50) est disposé dans un conduit qui est formé par un tuyau de vidange (42), un mélangeur statique (48) étant prévu en plus de préférence dans le tuyau de vidange (42) et permettant le mélange des composants de départ lors du passage dans le mélangeur (48).

3. Dispositif d'application de pâte conformément à la revendication n°1 ou n°2,
**caractérisé par le fait que**
le réservoir sous pression (3, 4) est composé de deux parties, les deux parties (3, 4) étant reliées solidairement par rivetage, par vissage et/ou par un écrou de raccord (6), une première partie (4) des deux parties du réservoir sous pression (3, 4) contenant de préférence la cartouche à deux composants (2) et la deuxième (3) des deux parties du réservoir sous pression (3, 4) contenant le cylindre creux (1).

4. Dispositif d'application de pâte conformément à l'une des revendications ci-dessus, **caractérisé par le fait que**
la cartouche à deux composants (2) est au moins localement une cartouche coaxiale, l'un des espaces intérieurs (16) étant cylindrique et interne dans la cartouche coaxiale et l'autre espace intérieur (18) étant cylindrique et englobant coaxialement l'espace intérieur (16) interne.

5. Dispositif d'application de pâte conformément à l'une des revendications ci-dessus, **caractérisé par le fait que**
le dispositif d'application de pâte présente un bouchon pour l'obturation des orifices de sortie (40) qui est à fixer avec un moyen de fixation du bouchon, notamment avec un filetage du bouchon, sur un contre-moyen de fixation (44) dans la zone des orifices de sortie (40), notamment sur un contre-filetage (44) dans la zone des orifices de sortie (40), le tuyau de vidange (42) étant à fixer de préférence sur le contre-moyen de fixation (44) qui présente, à cet effet, également un moyen de fixation (46) correspondant.

6. Dispositif d'application de pâte conformément à la revendication n°5,
**caractérisé par le fait que**
la fermeture est une clé (38) pour l'ouverture de la cartouche de gaz comprimé (30) et/ou pour le raccordement de la cartouche de gaz comprimé (30) au raccord (26) du cylindre creux (1), la fermeture étant à insérer comme clé (38) côté sol sur la cartouche de gaz comprimé (30) et, par conséquent, la cartouche de gaz comprimé (30) étant mobile, notamment pivotante et/ou déplaçable dans le sens longitudinal, avec le raccord (26) du cylindre creux (1) pour l'ouverture de la cartouche de gaz comprimé (30) et/ou le raccordement de la cartouche de gaz comprimé (30).

7. Dispositif d'application de pâte conformément à l'une des revendications ci-dessus, **caractérisé par le fait**
**que**, sur le raccord (26) du cylindre creux (1), est prévu un moyen de fixation de la cartouche de gaz comprimé (28), avec lequel la cartouche de gaz comprimé (30) est à fixer sur le raccord (26) de façon étanche à la pression et/ou que, sur le raccord (26) du cylindre creux (1), est prévu un montant de perçage (32) pour l'ouverture de la cartouche de gaz comprimé (30).

8. Dispositif d'application de pâte conformément à l'une des revendications ci-dessus, **caractérisé par le fait**
**qu'**un film de protection (24) est disposé entre le cylindre creux (1) et la cartouche à deux composants (2) qui ferme hermétiquement les espaces intérieurs (16, 18) de la cartouche à deux composants (2) sur la première face, le film de protection (24) étant de préférence collé, soudé ou agrafé sur la cartouche à deux composants (2).

9. Dispositif d'application de pâte conformément à l'une des revendications ci-dessus, **caractérisé par le fait**
**que** le réservoir sous pression (3, 4) est en aluminium, en zinc, en alliage en aluminium ou en acier.

10. Dispositif d'application de pâte conformément à l'une des revendications ci-dessus, **caractérisé par le fait**
**que** l'élément de vanne (50) commandé manuellement peut être commandé de l'extérieur par un élément de commande (54), de préférence par un levier (54) sur le tuyau de vidange (42) ou par une évacuation (54) sur une poignée (36) du dispositif d'application de pâte.

11. Dispositif d'application de pâte conformément à l'une des revendications ci-dessus, **caractérisé par le fait**
**qu'**au moins un orifice de ventilation (52) continu est prévu dans la paroi extérieure du cylindre creux (1), et ce dans la moitié qui est orientée vers la cartouche à deux composants (2) et dans la paroi du réservoir sous pression (3, 4) de sorte que la pression s'échappe du cylindre creux (1) quand le piston (8) est placé entre l'orifice de ventilation (52) et la cartouche à deux composants (2).

12. Dispositif d'application de pâte conformément à l'une des revendications ci-dessus, **caractérisé par le fait**
**que** la cartouche à deux composants (2), le cylindre creux (1) et le réservoir sous pression (3, 4) sont disposés dans un boîtier (34), la cartouche de gaz comprimé (30) étant de préférence également disposée dans le boîtier (34).

13. Dispositif d'application de pâte conformément à l'une des revendications ci-dessus, **caractérisé par le fait**
**que** les distances entre le réservoir sous pression (3, 4) et la paroi extérieure de la cartouche à deux composants (2) et entre le réservoir sous pression (3, 4) et la paroi extérieure du cylindre creux (1) sont inférieures à 100 µm, de préférence inférieures à 50 µm.

14. Dispositif d'application de pâte conformément à l'une des revendications ci-dessus, **caractérisé par le fait**
**que** le dispositif d'application de pâte est à maintenir avec une main et que l'élément de vanne (50) est à utiliser avec la même main.

15. Procédé pour le mélange et l'expulsion d'une pâte au moyen d'un dispositif d'application de pâte conformément à l'une des revendications n°1 à n°14, **caractérisé par** les étapes :
Un gaz comprimé est conduit de la cartouche de gaz comprimé (30) dans le cylindre creux (1) par le raccord (26) du cylindre creux (1), le réservoir sous pression (3, 4) absorbant la force du gaz comprimé exerçée sur les parois du cylindre creux (1) et le piston (8) étant avancé avec les coulisseaux (10) dans le cylindre creux (1) vers la cartouche à deux composants (2) par le gaz comprimé ;
les coulisseaux (10) avancés du piston (8) avancent les pistons d'alimentation (12, 14) dans les au moins deux espaces intérieurs (16, 18) de la cartouche à deux composants (2), les composants de départ étant expulsés des espaces intérieurs (16, 18) de la cartouche à deux composants (2) par les au moins deux orifices de sortie (40) des espaces intérieurs (16, 18),
le flux des composants de départ étant stoppé par un élément de vanne (50) fermé dans le sens d'écoulement derrière les orifices de sortie (40) et
l'élément de vanne (50) étant ouvert par une commande manuelle d'un élément de commande (54) de sorte que les composants de départ et/ou leur mélange circulent par l'élément de vanne (50) et que le mélange soit appliqué après un mélange des composants de départ.

16. Procédé conformément au point A, **caractérisé par le fait qu'**en l'absence de force exercée manuellement sur l'élément de commande (54), l'élément de vanne (50) est fermé par l'action de la force d'un élément de rappel (68), notamment d'un ressort élastique (68).

17. Procédé conformément à l'une des revendications n°15 ou n°16, **caractérisé par le fait que**
avant l'introduction du gaz comprimé est enlevé du dispositif d'application de pâte un bouchon qui obture les orifices de sortie (40) des espaces intérieurs (16, 18) de la cartouche à deux composants (2) et qu'un tuyau de vidange (42) dans lequel est disposé l'élément de vanne (50), est fixé devant les orifices de sortie (40) des espaces intérieurs (16, 18), la cartouche de gaz comprimé (30) étant de préférence ouverte après la fixation du tuyau de vidange (42) et le gaz comprimé étant conduit dans le cylindre creux (1), en particulier de préférence avec l'ouverture de la cartouche de gaz comprimé (30).
